# EUROPÄISCHE PATENTSCHRIFT

(11) **EP 0 922 771 B1**
(45) Veröffentlichungstag und Bekanntmachung des Hinweises auf die Patenterteilung: **12.04.2006**
(21) Anmeldenummer: 98120799.6
(22) Anmeldetag: 03.11.1998
(51) Int. Cl.: C12Q 1/70, C12Q 1/68

(54) **Verfahren zur Herstellung von Blutplasmaprodukten durch Ausschluss von Spenden mit hohen Parvo-B19-Virenkonzentrationen**
Method for producing blood plasma production through exclusion of donations having large concentrations of a Parvo B19 virus
Procédé de production de produits de plasma sanguin par exclusion des donations présentant une concentration élevée en virus Parvo B19

(30) Priorität: 28.11.1997 DE 19752898
(43) Veröffentlichungstag der Anmeldung: 16.06.1999
(73) Patentinhaber: ZLB Behring GmbH, 35041 Marburg (DE)
(72) Erfinder: Weimer, Thomas, Dr., 35075 Gladenbach (DE); Gröner, Albrecht, Dr., 64343 Seeheim (DE)

(56) Entgegenhaltungen:
- US-A- 5 538 848
- CARRIERE C ET AL: "RAPID AND SENSITIVE METHOD FOR THE DETECTION OF B19 VIRUS DNA USINGTHE POLYMERASE CHAIN REACTION WITH NESTED PRIMERS" JOURNAL OF VIROLOGICAL METHODS, AMSTERDAM, NL, Bd. 44, Nr. 2-3, 1993, Seiten 221-234, XP000982116 ISSN: 0166-0934
- HORNSLETH A ET AL: "Estimation of serum concentration of parvovirus B19 DNA by PCR in patients with chronic anaemia." RESEARCH IN VIROLOGY, Bd. 145, Nr. 6, 1994, Seiten 379-386, XP001094211 ISSN: 0923-2516
- FRICKHOFEN N ET AL: "POLYMERASE CHAIN REACTION FOR DETECTION OF PARVOVIRUS B19 IN IMMUNODEFICIENT PATIENTS WITH ANEMIA" BEHRING INSTITUTE MITTEILUNGEN, Nr. 85, 1990, Seiten 46-54, XP001094246 ISSN: 0301-0457
- SALDANHA J ET AL: "Collaborative study to assess the suitability of a proposed working reagent for human parvovirus B19 DNA detection in plasma pools by gene amplification techniques." VOX SANGUINIS, Bd. 73, Nr. 4, November 1997 (1997-11), Seiten 207-211, XP001094230 ISSN: 0042-9007
- LIVAK K J ET AL: "OLIGONUCLEOTIDES WITH FLUORESCENT DYES AT OPPOSITE ENDS PROVIDE A QUENCHED PROBE SYSTEM USEFUL FOR DETECTING PCR PRODUCT AND NUCLEIC ACID HYBRIDIZATION" GENOME RESEARCH, COLD SPRING HARBOR LABORATORY PRESS, US, Bd. 4, Nr. 6, 1. Juni 1995 (1995-06-01), Seiten 357-362, XP000522969 ISSN: 1088-9051
- GARTNER T ET AL: "PCR technology for HAV and parvovirus B19 plasmapool testing." VOX SANGUINIS, Bd. 74, Nr. SUPPL. 1, Juni 1998 (1998-06), Seite 1094 XP001095245 25th Congress of the International Society of Blood Transfusion;Oslo, Norway; June 27-July 2, 1998 ISSN: 0042-9007
- WEIMER THOMAS ET AL: "High-titer screening PCR: A successful strategy for reducing the parvovirus B19 load in plasma pools for fractionation." TRANSFUSION (BETHESDA), Bd. 41, Nr. 12, Dezember 2001 (2001-12), Seiten 1500-1504, XP001095087 ISSN: 0041-1132
- MCOMISH F ET AL: "DETECTION OF PARVOVIRUS B19 IN DONATED BLOOD: A MODEL SYSTEM FOR SCREENING BY POLYMERASE CHAIN REACTION" JOURNAL OF CLINICAL MICROBIOLOGY, WASHINGTON, DC, US, Bd. 31, Nr. 2, 1. Februar 1993 (1993-02-01), Seiten 323-328, XP000577180 ISSN: 0095-1137
- HARRIS S ET AL: "Optimisation of the polymerase chain reaction." BRITISH JOURNAL OF BIOMEDICAL SCIENCE, Bd. 54, Nr. 3, 1997, Seiten 166-173, XP001095226 ISSN: 0967-4845

## Beschreibung

Gegenstand der Erfindung ist ein Verfahren zur Herstellung von Blutplasmaprodukten, dadurch gekennzeichnet, dass alle Spenden, in denen mittels der Polymerasekettenreaktion (PCR) mehr als 10⁶ bis 10⁷ Genomäquivalente/ml von Parvovirus B19, nachgewiesen werden, ausgesondert werden, wobei die Empfindlichkeit der PCR so eingestellt wird, daß Parvovirus B19-DNA nur in solchen Spenden nachgewiesen wird, deren DNA-Gehalt größer als 10⁶ bis 10⁷ Genomäquivalente/ml ist.

Es ist bekannt, daß virale Verunreinigungen, die in Plasmaproteinlösungen enthalten sind, auch in die daraus hergestellten Plasmaprodukte gelangen und dann zu Infektionen bei Patienten führen können, denen derartige Blutplasmaprodukte verabreicht werden. Um dieser Gefahr vorzubeugen, sind vielfältige Sicherheitsmaßnahmen entwickelt worden. Unter ihnen haben die Untersuchung des Spenders auf bestimmte Virusinfektionen sowie die Inaktivierung und Eliminierung von Viren vor, während und nach der Gewinnung der Plasmaprodukte aus dem Spenderblut ganz besondere Bedeutung erhalten.

Häufig ist Spenderblut mit Parvovirus B19 verunreinigt. Parvoviren führen zwar nur in Ausnahmefällen zu ernsten Erkrankungen, jedoch sollte aus Sicherheitsgründen Spenderblut, das mit hohen Titern von Parvoviren belastet ist, nicht zur Gewinnung von Plasmaprodukten herangezogen werden. Niedrige Titer an Parvoviren sind demgegenüber im Spenderblut tolerierbar, weil physikalische und chemische Methoden zur Verfügung stehen, mit denen geringe Restmengen an Viren im zu verarbeitenden Plasma bei der Gewinnung von Plasmaproteinen entfernt werden können.

Wünschenswert ist es deshalb, Spenderblut mit überdurchschnittlich hohen Konzentrationen an Parvo-B19-Viren (B19V) rechtzeitig zu erkennen und von der Verarbeitung zu Plasmaprodukten auszuschließen.

Es stellte sich deshalb die Aufgabe, ein Nachweisverfahren für hochtitrige Plasmaproteinlösungen zu entwickeln, das nur das Vorhandensein überdurchschnittlich großer Mengen von B19V zu erkennen gibt, hingegen beim Vorhandensein geringer Mengen von B19V keine Reaktion zeigt.

Die Polymerasekettenreaktion (PCR) ist bekanntlich eine sehr wirkungsvolle Methode zum Nachweis geringer Mengen einer bekannten Nukleinsäure-Sequenz in einer Probe (Erlich H.A., Gelfand D., Sninsky J.J. (1991), Science, 252, pp. 1643-1651; PCR Protocols. Current methods and applications (1993) edited by B.A. White, Humana Press, Totowa, New Jersey, ISBN 0-89603-244-2). Wenn die Sequenz der Virus-DNA bereits bekannt ist, kann ein Primerpaar synthetisiert werden, das zu Regionen auf einander gegenüberliegenden Einzelsträngen komplementär ist und die gesuchte DNA-Sequenz flankiert. Unter den an sich bekannten Bedingungen einer PCR lassen sich dann in vitro durch Aufeinanderfolge von in der Regel mehr als 30 Reaktionszyklen große Mengen einer spezifischen DNA amplifizieren. Durch die PCR-Zyklen wird ein DNA Fragment einer spezifischen Größe, das sich aus den Längen der beiden Primer plus der Länge der Virus-DNA zwischen Ihnen zusammensetzt, nur dann amplifizieren, wenn die gesuchte Virus-DNA in der Probe vorhanden ist.

Die PCR-Technik ist so empfindlich, daß damit außerordentlich geringe Mengen einer DNA mit großer Sicherheit nachgewiesen werden können. Die große Empfindlichkeit der PCR führt auch dann zu positiven Ergebnissen, wenn die nachzuweisende Virus-DNA in einer Plasmaprobe in so geringen Mengen vorhanden ist, daß dadurch die praktische Brauchbarkeit der Spende, aus der die Probe stammt, zur Herstellung von Blutplasmaprodukten nicht beeinträchtigt ist. Zum Nachweis der Parvovirus B19-DNA ist beispielsweise die übliche qualitative PCR-Technik nicht geeignet, da zwischen hohen und niedrigen Parvovirus B19-Konzentrationen nicht unterschieden werden kann und auch Plasmaproben mit niedrigem Parvovirus B19-Titer als stark virenbelastet erscheinen.

Carriere, C. et al. (1993), J. Virol. Methods, 44, pp. 221-234 beschreibt ein Verfahren zum Nachweis geringer B19V-Mengen mittels PCR.

Es wurde nun gefunden daß das Verfahren zum Nachweis hoher Konzentrationen von B19V im Blutplasma und/oder Blutserum mittels der Polymerasekettenreaktion für die praktische Anwendung in sinnvoller Weise abgewandelt werden kann, wenn die Empfindlichkeit der Polymerasekettenreaktion durch die Anwendung suboptimaler Nukleinsäure-Extraktions-, Amplifikations- oder Detektionsbedingungen gedrosselt wird.

Dieses Verfahren ist zum Nachweis hoher Konzentrationen von Nukleinsäuren von B19V geeignet. Erfindungsgemäß wird die Empfindlichkeit der PCR für den Nachweis der Nukleinsäuren von B19V so stark gedrosselt, daß die Nukleinsäuren der B19V nur noch in Proben nachgewiesen werden können, deren DNA-Gehalt größer als 10⁶ bis 10⁷ Genomäquivalente ist. Proben mit einem geringeren B19V DNA-Gehalt werden nicht mehr als positiv erkannt und können der weiteren Verarbeitung zu Plasmaprodukten zugeführt werden, wenn etwa noch vorhandene geringe B19V-Mengen während des Produktionsverfahrens entfernt werden.

Zur Drosselung der Empfindlichkeit der PCR stehen mehrere Verfahren zur Verfügung. So kann beispielsweise die Nukleinsäure-Extraktion unter Bedingungen erfolgen, bei denen nur geringe Mengen an Nukleinsäuren isoliert werden oder es kann mit hochverdünnten Lösungen gearbeitet werden, in denen nur geringe Mengen Nukleinsäuren enthalten sind. Suboptimale Amplifikationsbedingungen können z.B. dadurch eingestellt werden, daß die Anlagerung der Primer an den DNA-Einzelstrang (Annealing) und die Amplifikation bei ungewöhnlich niedrigen Temperaturen erfolgt und die Anzahl der PCR-Zyklen begrenzt wird oder die Amplifikation "gebremst wird" durch Reduktion der Primerkonzentration unter dem optimalen Bereich. Schließlich ist es auch möglich, die Detektionsbedingungen so auszuwählen, daß nur besonders hohe Konzentrationen an B19V-DNA erkannt werden.

Bekanntlich beginnt die übliche PCR mit einer bei 90°C oder höher für zum Beispiel 20 Sekunden bis 1 Minute durchgeführten Denaturierung der aus der Untersuchungsprobe extrahierten Nukleinsäuren.

Diese werden dabei in Einzelstränge zerlegt, an die normalerweise die Primer bei einer Temperatur von 45 bis 65°C angelagert werden. Anschließend erfolgt dann die Amplifikation bei 72°C.

Demgegenüber wird erfindungsgemäß das Annealing und/oder die Amplifikation bei einer Temperatur von etwa 52°C durchgeführt. Außerdem wird die Zahl der PCR-Zyklen auf 30 begrenzt. Damit wird erreicht, daß die Reaktionsgeschwindigkeit der PCR erheblich gedrosselt und durch die Verminderung der Zyklen die Menge der gebildeten B19V-DNA erheblich vermindert wird.

Als Primer werden beispielsweise die folgenden Oligonukleotide eingesetzt.

SEQ ID NO. 1: 5' ATG GGC CGC CAA GTA CAG GAA A 3'

SEQ ID NO. 2: 5' CAG GCA CAG CTA CAC TTC CAG GC 3'

Es hat sich bei dem erfindungsgemäßen Verfahren nun als besonders vorteilhaft erwiesen, den Nachweis des amplizierten B19V DNA-Abschnitts mittels einer Sonde zu führen, die zwei Fluoreszenzfarbstoffe trägt. Diese markierte Sonde, die zwischen den beiden durch die vorstehend genannten Sequenzen charakterisierten Primern an die DNA angelagert ist, weist dann z. B. folgende Sequenz auf:

SEQ ID NO. 3: 5' FAM-TGG TGG TCT GGG ATG AAG GTA TTA TT-
TAMRA3'

Diese Sequenz kann unter der Bezeichnung TaqMan® Sonde im Handel erworben werden und ist zur Verwendung im 5'-Nuklease-Assay, dem TaqMan Assay, bestimmt. Im einzelnen ist diese Methode beschrieben von Livak K.J., Flood S.J.A., Marmaro J., Giusti W., Deetz K., Oligonucleotides with fluorescent dyes at opposite ends provide a quenched probe system useful for detecting PCR product and nucleic acid hybridization. PCR Method and Appl. 1995; 4:357-362.

Die besondere Eigenschaft dieser Sonde besteht darin, daß die Fluoreszenz des am 5'-Ende der Sonde befestigten Farbstoffes (FAM), des Reporters, durch die Nähe des am 3'-Ende des Primers angeordneten zweiten Fluoreszenzfarbstoffes (TAMRA), des Quenchers, reduziert wird.

Im Verlauf der Amplifikation wird nun unter der Einwirkung einer thermostabilen DNA-Polymerase, vorzugsweise der Taq DNA Polymerase, der neue DNA-Strang ausgebildet. Dabei verdrängt die DNA-Polymerase die Sonde nicht nur vom Einzelstrang, sondern zerlegt ihn mittels ihrer endonukleolytischen Aktivität und setzt dabei die beiden Fluoreszenzfarbstoffe frei. Die Fluoreszenz des Reporterfarbstoffes wird jetzt nicht mehr durch den Quencherfarbstoff unterdrückt und steigt an. Mißt man nun mit einem Fluoreszenzspektrometer die Fluoreszenz bei der Reporter- und bei der Quencherwellenlänge (518 nm für FAM und 582 nm für TAMRA), dann kann der Quotient aus Reporter- und Quenchwert gebildet werden (RQ). Der Mittelwert der Quotienten mehrerer Negativkontrollen (RQ⁻) wird davon abgezogen und der errechnete Wert als ΔRQ bezeichnet. Die Methode zur Erkennung der DNA-Bildung bei der PCR durch die Verwendung eines mit zwei Fluoreszenzfarbstoffen markierten Primers ist in der internationalen Patentanmeldung WO 92/02638 beschrieben.

Wird dieses Verfahren zum Nachweis der Parvovirus B19 DNA in dem erfindungsgemäßen PCR-Verfahren mit gedrosselter Empfindlichkeit eingesetzt, dann werden Proben mit einem ΔRQ-Wert größer oder gleich 0,7 als positiv bewertet und aussortiert. Proben mit einem niedrigen ΔRQ-Wert enthalten geringe Mengen an Parvovirus B19 DNA (< 10⁶ bis 10⁷ Genomäquivalente/ml).

Die Erfindung wird durch das folgende Beispiel erläutert:

### Beispiel

### A Nukleinsäure-Extraktion

Die Nukleinsäure-Extraktion wird mit einer verbesserten Version der von Ishizawa et al. (Ishizawa M., Kobayashi Y., Miyamura T., Matsuma S.: Simple procedure of DNA isolation from human serum. Nucl. Acids Res. 1991; 19:5792) beschriebenen Methode durchgeführt. Dazu werden 100 µl Plasma oder Serum mit 300 µl Extraktionspuffer (6 M Natriumjodid, 13 mM EDTA, 0,5% Sarcosyl, 26 mM Tris-HCl, pH 8,0, 33 µg/ml Glykogen, 77 µg/ml Hefe tRNA) gemischt und 15 Minuten bei 60 ± 2°C inkubiert. Durch Zugabe von 400 µl Isopropanol werden die Nukleinsäuren für 20 Minuten bei Raumtemperatur gefällt. Es schließt sich eine 10-minütige Zentrifugation bei 13.000 bis 14.000 UpM in einer Tischzentrifuge zur Pelletierung der Nukleinsäuren an. Der Überstand wird verworfen, das Pellet (Niederschlag) mit 1 ml 40% Isopropanol gewaschen, getrocknet und in 20 µl H₂O resuspendiert.

### B Amplifikation

Die Amplifikation der B19V DNA im NS1-Gen wird durch suboptimale Annealing- und Verlängerungstemperaturen so eingestellt, daß nur solche Proben positiv werden, deren DNA-Gehalt größer 10⁶ bis 10⁷ Genomäquivalente/ml beträgt. Zu 10 µl der extrahierten DNA werden 40 µl Master Mix (5 µl 10 x PCR-Puffer (Perkin-Elmer Katalog 1995/96 PCR-Systems, Reagents and Consumables), 3 µl 25 mM MgCl₂, 4 µl 2,5 mM Deoxynukleosidtriphosphat, jeweils 4 µl der Oligonukleotide der SEQ ID NO. 1 und der SEQ ID NO. 2 (je 10 pmol/µl), 0,3 µl des Oligonukleotids der SEQ ID NO. 3 (10 pmol/µl), 0,25 µl Taq DNA Polymerase (1,25 Units; Perkin-Elmer Katalog 1995196 PCR-Systems, Reagents and Consumables), 11,45 µl H₂O) pipettiert, gemischt und folgenden Thermozyklen unterworfen:
1. Initiale Denaturierung für 1 Minute bei 90°C
2. 30 Zyklen, jeweils 28 Sekunden bei 94°C Denaturierung und 1 Minute bei 52°C Annealing und Verlängerung
3. Kühlen bei 4°C bis zur Auswertung.

### C Auswertung

Die PCR-Reaktion wird in einem Fluoreszenzspektrometer ausgewertet. Dazu wird die Fluoreszenz bei der Reporter- und Quenchwellenlänge (518 nm für FAM oder 582 nm für TAMRA) gemessen und der jeweilige Quotient aus Reporter- und Quencherwert gebildet (RQ). Der Mittelwert der Quotienten dreier Negativkontrollen (RQ⁻) wird davon abgezogen, der errechnete Wert wird als ΔRQ bezeichnet. Proben mit einem ΔRQ größer oder gleich 0,7 werden als positiv gewertet, Proben kleiner 0,7 als negativ. Dadurch werden nur solche Proben als positiv bewertet, deren DNA-Gehalt größer 10⁶ bis 10⁷ Genomäquivalente/ml beträgt.

### Sequenzprotokoll:

| Allgemeine Angaben | |
|---|---|
| Anmelder: | Centeon Pharma GmbH |
| | Emil-von-Behring-Strasse 76 35041 Marburg |
| | Bundesrepublik Deutschland |
| | Tel.: (0 64 21) 39-20 69 |
| | Fax: (0 64 21) 39-45 58 |
| | |
| Bezeichnung der Erfindung: | Verfahren zum Nachweis hochtitriger Plasmaproteinlösungen mittels der Polymerasekettenreaktion |
| | |
| Anzahl der Sequenzen: | 3 |
| | |
| Zustellanschrift: | Centeon Pharma GmbH |
| | P. O. Box 12 30 |
| | 35002 Marburg |
| | Germany |
| | |
| Computerlesbare Fassung: | |
| Datenträger: | Diskette |
| Computer: | IBM PC compatible |
| Operating System: | PC-DOS / MS-DOS |
| Software: | Microsoft Word 6.0 |

### Angaben zu SEQ ID NO. 1:

| | |
|---|---|
| Länge: | 22 Basenpaare |
| Art: | Oligonucleotid |
| Stangform: | Einzelstrang |
| Topologie: | linear |
| Herkunft: | Chemische Synthese |
| Merkmal: | Primer für die PCR einer Parvovirus B 19 Sequenz |
| Name: | Oligo 1 |

### Sequenzbeschreibung:

5´ ATG GGC CGC CAA CAG GAA A3´

### Angaben zur SEQ ID NO. 2:

| | |
|---|---|
| Länge: | 23 Basenpaare |
| Art: | Oligonucleotid |
| Stangform: | Einzelstrang |
| Topologie: | linear |
| Herkunft: | Chemische Synthese |
| Merkmal: | Primer für die PCR einer Parvovirus B 19-DNA-Sequenz |
| Name: | Oligo 2 |

### Sequenzbeschreibung:

5' CAG GCA CAG CTA CAC TTC CAC GC3'

### Angaben zur SEQ ID NO. 3:

| | |
|---|---|
| Länge: | 26 Basenpaare mit zwei endständigen Fluoreszenzfarbstoffen |
| Art: | Oligonukleotid |
| Strangform: | Einzelstrang |
| Topologie: | linear |
| Herkunft: | Chemische Synthese |
| Merkmal: | TaqMan® Sonde zur Verwendung im |
| | 5'-Nuklease-Assay |
| Name: | Oligo 3 |

### Sequenzbeschreibung:

5´ FAM-TGG-TGG-TCT-GGG-ATG-AAG-GTA-TTA-TT-TAMRA3´

FAM und TAMRA sind Fluoreszenzfarbstoffe

## Patentansprüche

1. Verfahren zur Herstellung von Blutplasmaprodukten, **dadurch gekennzeichnet, dass** alle Spenden, in denen mittels der Polymerasekettenreaktion (PCR) mehr als 10⁶ bis 10⁷ Genomäquivalente/ml von Parvovirus B19, nachgewiesen werden, ausgesondert werden, wobei die Empfindlichkeit der PCR so eingestellt wird, daß Parvovirus B19-DNA nur in solchen Spenden nachgewiesen wird, deren DNA-Gehalt größer als 10⁶ bis 10⁷ Genomäquivalente-ml ist.

2. Verfahren nach Anspruch 1. **dadurch gekennzeichnet, daß** die Empfindlichkeit der PCR **dadurch** eingestellt wird, daß die Anlagerung der Primer an den DNA-Einzelstrang (Annealing) sowie die Amplifikation bei einer Temperatur von etwa 52°C stattfinden und nicht mehr als 30 Zyklen durchgeführt werden.

3. Verfahren nach mindestens einem der Ansprüche 1 bis 2, **dadurch gekennzeichnet, daß** die Empfindlichkeit der PCR **dadurch** eingestellt wird, daß mit hochverdQnnten Proben gearbeitet wird .

4. Verfahren nach mindestens einem der Ansprüche 1 bis 3, **dadurch gekennzeichnet, daß** der amplifizierte Parvovirus-DNA-Abschnitt mit einer zusätzlichen Sonde nachgewiesen wird, die zwei Fluoreszenzfarbstoffe trägt, wobei die Fluoreszenz des einen Farbstoffes (Reporter) durch die Nähe des zweiten Farbstoffes (Quencher) vermindert ist, solange der zusätzliche Primer noch intakt ist, und die Fluoreszenz des Reporters ansteigt, sobald der zusätzliche Primer durch eine thermostabile DNA-Polymerase verdrängt und durch deren endonukleolytische.

5. Verfahren nach mindestens einem der Ansprüche 1 bis 4. **dadurch gekennzeichnet daß** als thermostabile DNA-Polymerase bevorzugt die Taq-DNA-Polymerase eingesetzt wird.

## Claims

1. Process for preparing blood plasma products, **characterized in that** all donations in which more than 10⁶ to 10⁷ genome equivalents/ml of parvovirus B19 can be detected by polymerase chain reaction (PCR) are rejected, the sensitivity of the PCR being adjusted such that the parvovirus B19 DNA can only be detected in those samples whose DNA content is greater than 10⁶ to 10⁷ genome equivalents/ml.

2. Process according to Claim 1, **characterized in that** the sensitivity of the PCR is adjusted by the annealing of the primers to the DNA single strand and the amplification taking place at a temperature of about 52°C and not more than 30 cycles being carried out.

3. Process according to at least one of Claims 1 and 2, **characterized in that** the sensitivity of the PCR is established by working with highly dilute samples.

4. Process according to at least one of Claims 1 to 3, **characterized in that** the amplified parvovirus DNA section is detected by an additional probe which carries two fluorescent dyes, the fluorescence of one dye (reporter) being reduced by the vicinity of the second dye (quencher) as long as the additional primer is still intact, and the fluorescence of the reporter increasing as soon as the additional primer is displaced by a thermostable DNA polymerase and the fluorescent dyes are released by means of its endonucleolytic activity.

5. Process according to at least one of Claims 1 to 4, **characterized in that** the thermostable DNA polymerase employed is preferably the Taq DNA polymerase.

## Revendications

1. Procédé pour la production de produits de type plasma sanguin, **caractérisé en ce qu'**on exclut tous les dons chez lesquels sont détectés par la réaction d'amplification par polymérisation en chaîne (PCR) plus de 10⁶ à 10⁷ équivalents génomiques/ml de parvovirus B 19, la sensibilité de la PCR étant ajustée de manière que l'ADN de parvovirus B 19 ne soit détecté que chez les donneurs dont la teneur en ADN est supérieure à 10⁶-10⁷ équivalents génomiques/ml.

2. Procédé selon la revendication 1, **caractérisé en ce que** la sensibilité de la PCR est ajustée de manière que l'hybridation des amorces avec l'ADN simple brin (*annealing*) ainsi que l'amplification aient lieu à une température d'environ 52°C et que 30 cycles au maximum soient effectués.

3. Procédé selon au moins l'une des revendications 1 et 2, **caractérisé en ce qu'**on ajuste la sensibilité de la PCR en opérant avec des échantillons fortement dilués.

4. Procédé selon au moins l'une des revendications 1 à 3, **caractérisé en ce que** le segment amplifié d'ADN de parvovirus est détecté avec une sonde supplémentaire qui porte deux colorants fluorescents, la fluorescence d'un colorant (rapporteur) étant diminuée par la proximité du second colorant (extincteur) lorsque l'amorce supplémentaire est encore intacte, et la fluorescence du rapporteur augmentant dès que l'amorce supplémentaire est déplacée par une ADN polymérase thermostable et par son activité endonucléolytique.

5. Procédé selon au moins l'une des revendications 1 à 4, **caractérisé en ce qu'**en tant qu'ADN polymérase thermostable on utilise de préférence l'ADN polymérase Taq.
